# EUROPÄISCHE PATENTSCHRIFT

(11) **EP 1 038 509 B1**
(45) Veröffentlichungstag und Bekanntmachung des Hinweises auf die Patenterteilung: **26.01.2005**
(21) Anmeldenummer: 00810157.8
(22) Anmeldetag: 24.02.2000
(51) Int. Cl.: A61F 2/46, A61B 17/16, A61B 17/32, A61B 10/00

(54) **Instrument und Instrumentensatz zum Einbringen eines osteochondralen Transplantats**
Instrument and set of instruments for inserting an osteochondral graft
Instrument et jeu d'instruments pour l'introduction d'un greffon ostéochondral

(30) Priorität: 23.03.1999 EP 99810259
(43) Veröffentlichungstag der Anmeldung: 27.09.2000
(73) Patentinhaber: Zimmer GmbH, 8404 Winterthur (CH)
(72) Erfinder: Frei, Heribert, 8404 Winterhur (CH); Roder, Daniel, 2533 Evilard (CH); Jakob, Roland P.,prof. Dr. med., 1787 Môtier (CH)
(74) Vertreter: Manitz, Finsterwald & Partner Gbr

(56) Entgegenhaltungen:
- EP-A- 0 470 393
- EP-A- 0 824 893
- WO-A-96/27333
- WO-A-98/34569
- DE-A- 2 311 521
- DE-A- 19 503 504
- FR-A- 2 398 490
- FR-A- 2 716 375
- US-A- 5 632 747
- US-A- 5 718 707

## Beschreibung

Die Erfindung betrifft ein Instrument sowie einen Instrumentensatz zum Einbringen eines osteochondralen Transplantats bzw. eines entsprechenden in vitro hergestellten Implantats gemäss dem Oberbegriff des jeweiligen unabhängigen Patentanspruchs 1 bzw. 8.

Die Behandlung von Knorpeldefekten mit Hilfe der Transplantation von osteochondralen Transplantaten ist eine Art der Behandlung, die in neuerer Zeit immer häufiger und mit Erfolg angewandt wird. Im wesentlichen läuft diese Art der Behandlung derart ab, dass zunächst an der Defektstelle eine oder mehrere Vertiefungen (z.B. Sacklochbohrungen) erstellt werden, z.B. mittels eines geeigneten Bohrers. Anschliessend werden an einer Erntestelle (vorzugsweise ist die Erntestelle mechanisch mehr oder weniger unbelastet) Transplantate wie z.B. Knochenstifte (sogenannte "pegs"), die eine intakte Knorpelschicht aufweisen, entnommen. Diese Knochenstifte werden dann an der Defektstelle in die erstellte Sacklochbohrung eingebracht. Der Peg verwächst über die Zeit mit intaktem Knorpel und Knochen. Je nach Grösse des Defekts können an der Defektstelle auch mehrere Sacklochbohrungen an der Defektstelle erstellt werden und dementsprechend mehrere Pegs eingesetzt werden, weil die lateralen Abstände (Lücken) zwischen dem Knorpelbelag der Transplantate und dem intakten Knorpel um die Defektstelle herum nicht zu gross werden dürfen. Anstelle des Transplantats kann auch ein in vitro gezüchtetes Knorpelimplantat an der Defektstelle implantiert werden. Der umliegende intakte Knorpel verwächst in diesem Fall mit dem in vitro gezüchteten Knorpel.

Das Einbringen eines Pegs - im folgenden wird der Ausdruck "Peg" immer sowohl für ein Transplantat wie auch für ein Implantat verwendet, erfolgt in bekannter Weise derart, dass der in einem Kanal eines entsprechenden Instruments befindliche Peg mit Hilfe eines Stempels aus dem Instrument heraus in die Sacklochbohrung hinein gedrückt wird. Hierzu wird die Stirnfläche des Stempels gegen die dem Stempel zugewandte Oberfläche des Pegs gedrückt und der Stempel vorgeschoben - der Stempel befindet sich also bei diesem Vorgang in körperlichem Kontakt mit der Knorpeloberfläche des Pegs und übt Kraft auf diese Knorpeloberfläche aus.

Hinsichtlich des diesbezüglichen Standes der Technik wird z.B. auf WO-A-96127333 und DE-A-19503504 verwiesen.

Knorpelzellen - Chondrozyten - sind empfindlich auf zu hohe Flächenpressungen, also auf zu hohe Kräfte, die auf zu kleine Flächen wirken. Wenn eine für die Knorpelzellen typische zulässige Flächenpressung überschritten wird, so können die Knorpelzellen bzw. das Knorpelgewebe beschädigt werden. Geschädigter Knorpel regeneriert sich kaum. Knorpel ist darüberhinaus auch empfindlich im Hinblick auf Deformation, das heisst, bei ungleichmässiger Beaufschlagung mit Kräften erfolgt eine Knorpeldeformation (wodurch der Knorpel ebenfalls geschädigt werden kann).

Andererseits ist es so, dass Knorpeloberflächen von Pegs gewöhlich konvex sind, selten konkav, fast nie eben, oft sogar schief (geneigt). Infolgedessen sind die Knorpeloberfläche und die in der Regel ebene Stirnfläche des Stempels inkongruent und es ergibt sich oft eine punkt- oder linienfömige Auflage, sodass sich am Ort der Auflage eine hohe Flächenpressung ergibt. Hinzu kommt noch, dass die Sacklochbohrung an der Defektstelle gegenüber dem Peg ein geringes Untermass aufweist, sodass der Peg nach dem Einbringen in einem Pressitz in der Sacklochbohrung sitzt. Da beim Einbringen des Pegs in die Sacklochbohrung der Stempel durch leichte Schläge auf das proximale Ende des Stempels vorgeschoben wird, muss jedesmal die Haftreibung zwischen dem Peg und der Wand der Sacklochbohrung überwunden werden (stop-and-go). Dies führt zu einer noch höheren Belastung der Knorpeloberfläche und kann Schädigungen des Knorpels zur Folge haben.

Durch die Reibung zwischen Stempel und Knorpeloberfläche treten ausser den Belastungen infolge des Schlagimpulses noch Scherbelastungen auf, welche die Knorpelzellen bzw. das Knorpelgewebe schädigen können. Dies ist ebenfalls eine Folge der Inkongruenz von Knorpeloberfläche und Stirnfläche des Stempels. Da geschädigter Knorpel sich kaum regeneriert, ist in solchen Fällen der Erfolg der Transplantation - und damit der Nutzen für den Patienten - gering. In einigen Fällen erweist sich sogar das Einbringen von Pegs mit schiefer (geneigter) Oberfläche als unmöglich.

Hier will die vorliegende Erfindung Abhilfe schaffen. Es ist daher Aufgabe der Erfindung, ein Instrument bzw. einen Instrumentensatz vorzuschlagen, mit welchem es möglich ist, ein osteochondrales Transplantat oder ein entsprechend in vitro hergestelltes Implantat unabhängig von der Form der Knorpeloberfläche in eine an der Defektstelle erstellte Vertiefung (z.B. die erwähnte Sacklochbohrung) einzutreiben, ohne dabei die Knorpeloberfläche des Transplantats bzw. Implantats zu schädigen oder zu deformieren (wodurch der Knorpel ebenfalls geschädigt werden kann).

Diese Aufgabe wird durch das Instrument bzw. den Instrumentensatz gelöst, wie es durch die Merkmale des jeweiligen unabhängigen Patentanspruchs 1 bzw. 8 charakterisiert ist. Besonders vorteilhafte Ausgestaltungen ergeben sich aus den Merkmalen der abhängigen Patentansprüche.

Dabei soll vorausgeschickt werden, dass unter einem "Stempel" im Sinne der vorliegenden Erfindung nicht nur ein körperlicher Stempel verstanden werden soll, sondern generell ein Mittel zum Ausüben von Kräften bzw. von Druck auf das Puffermedium. Dies kann z.B. auch mit Hilfe einer Pumpe bewirkt werden, ohne dass dazu ein körperlicher Stempel mit dem Puffermedium in Berührung kommt.

Erfindungsgemäss ist bei dem Instrument zwischen der dem Transplantat bzw. Implantat (im folgenden wird der Einfachheit halber stets nur noch auf das Transplantat Bezug genommen) zugewandten Stirnfläche des Stempels und der dem Stempel zugewandten Oberfläche des Transplantats ein Raum zur Aufnahme eines Puffermediums mit einer hohen Formanpassungsfähigkeit vorgesehen, sodass sich das Puffermedium an der Grenze zur Oberfläche des Transplantats optimal an die Form dieser Oberfläche anpassen kann. Ferner weist das Puffermedium eine Elastizität auf, die so bemessen ist, dass beim Herausdrücken des Transplantats kein körperlicher Kontakt zwischen dem Stempel und dem Transplantat besteht. Durch die optimale Anpassung des Puffermediums an die Form der Oberfläche des Transplantats wird erreicht, dass die Flächenpressung so gering wie möglich ist (die Kräfte werden in etwa homogen über die gesamte Oberfläche des Transplantats übertragen). Andererseits wird durch die Elastizität des Puffermediums ein mechanischer Kontakt zwischen dem Stempel und dem Transplantat vermieden, sodass es nicht zu einer inkongruenten Auflage des Stempels auf der Oberfläche des Transplantats kommen kann. Somit können Transplantate bzw. Implantate mit einer beliebigen Oberfläche in die Vertiefungen (z.B. Sacklochbohrungen) an der Defektstelle eingetrieben werden, ohne dass dabei die Transplantatoberfläche beschädigt oder deformiert (und dadurch möglicherweise geschädigt) werden kann.

Bei einem vorteilhaften Ausführungsbeispiel ist der Stempel mit Anschlagmitteln versehen, welche eine Endstellung des Stempels definieren und ein weiteres Vorschieben des Stempels über die Endstellung hinaus verhindern. Dadurch kann einerseits verhindert werden, dass das Transplantat über eine gewünschte Endstellung hinaus weiter mit Kräften beaufschlagt wird. Ist das Transplantat nämlich z.B. bis zu einer gewünschten Tiefe in die Vertiefung an der Defektstelle eingetrieben worden (nicht notwendigerweise bis zum Boden der Vertiefung), so soll es nicht möglich sein, durch weiteres Aufbringen von Kräften das Transplantat weiter in die Vertiefung einzutreiben bzw. es soll nicht möglich sein, die Oberfläche des Transplantats durch Aufbringen von zu hohen Flächenpressungen zu beschädigen, wie sie dann auftreten können, wenn das Transplantat bereits bis an den Boden der Vertiefung eingetrieben worden ist, aber dennoch das Transplantat durch weiteres Vorschieben des Stempels mit immer höheren Flächenpressungen beaufschlagt wird. Hier sorgt der (vorzugsweise einstellbare) Anschlag für Abhilfe, indem er ein weiteres Vorschieben des Stempels verhindert.

Bei einem weiteren vorteilhaften Ausführungsbeispiel ist das Puffermedium ein Fluid, insbesondere eine Flüssigkeit, und der Stempel ist mit einem Dichtmittel versehen ist, welches den Kanal abdichtet und beim Vorschieben des Stempels in distaler Richtung ein Zurückströmen von Fluid in proximaler Richtung verhindert. Flüssigkeiten sind besonders bevorzugt, weil sie einerseits im wesentlichen inkompressibel sind und somit einen körperlichen Kontakt zwischen dem Stempel und der Oberfläche des Transplantats zuverlässig verhindern, sich aber andererseits optimal an die Form der Oberfläche des Transplantats anpassen und somit für eine minimale Flächenpressung besorgt sind. Als Flüssigkeiten kommen vorzugsweise körperverträgliche Flüssigkeiten wie z.B. eine Ringer-Lösung (eine Spüllösung, die im Operationssaal gewöhnlich eingesetzt wird) oder eine Natriumchloridlösung (Kochsalzlösung) zum Einsatz. Beim Vorschieben des Stempels wird die Flüssigkeit vor dem Stempel hergetrieben und überträgt die Kraft optimal auf die Oberfläche des Transplantats, unabhängig von der Form der Transplantatoberfläche.

Bei einer Weiterbildung dieses Ausführungsbeispiels ist das Dichtmittel als O-Ring ausgebildet, welcher im distalen Endbereich des Stempels angeordnet ist, und zwar proximal zu der dem Transplantat zugewandten Stirnfläche des Stempels. Dadurch steht die gesamte Stirnfläche des Stempels zur Verfügung für die Übertragung der Kräfte auf die Flüssigkeit, welche ihrerseits für die optimale Übertragung der Kräfte auf die Transplantatoberfläche besorgt ist.

Bei einer Weiterbildung dieses Ausführungsbeispiels umfasst das Instrument eine Hülse, die an ihrem distalen Ende mit einer Schneide versehen ist. In der Wand der Hülse unmittelbar anschliessend an das proximale Ende der Schneide sind Durchtrittsöffnungen vorgesehen, welche ein Austreten des Fluids ermöglichen, sobald beim Herausdrücken des Transplantats das proximale Ende des Transplantats die Durchtrittsöffnungen freigibt. Auf diese Weise kann erreicht werden, dass das Transplantat nach dem Passieren der Durchtrittsöffnungen nicht mehr oder nur noch minimal weiter eingetrieben werden kann, weil die Flüssigkeit durch die Durchtrittsöffnungen entweichen kann und somit die Kräfte nicht mehr auf das Transplantat übertragen werden. Dies spürt der operierende Arzt an der plötzlichen Abnahme des Widerstandes und weiss dann, dass jetzt das Transplantat bis zu der gewünschten Tiefe eingetrieben ist. Auf diese Weise kann also eine Art nicht-mechanischer "Tiefenanschlag" realisiert werden. Zusätzliche können durchaus noch mechanischen Anschlagmitteln (siehe oben) vorgesehen sein, um zuverlässig einen körperlichen Kontakt zwischen dem Stempel und dem Transplantat zu vermeiden, sollte einmal der Stempel trotz Erreichen des nicht-mechanischen "Tiefenanschlags" weiter vorgeschoben werden.

Bei einem weiteren besonders vorteilhaften Ausführungsbeispiel des Instruments ist der Stempel an seinem proximalen Ende mit einem Antrieb koppelbar, welcher einen kontinuierlichen Vorschub des Stempels bewirkt. Wenn nämlich das Transplantat - wie eingangs beschrieben - jeweils immer nur mit Hilfe eines Impulsschlags ein Stück weit eingetrieben wird, anschliessend aber wieder zur Ruhe kommt, bevor es mit dem nächsten Impulsschlag um ein weiteres Stück eingetrieben wird, so muss jedesmal die Haftreibung überwunden werden. Wird hingegen das Transplantat kontinuierlich eingetrieben, so ist nur einmal (nämlich zu Beginn) die Haftreibung zu überwinden und anschliessend muss nur noch die Gleitreibung überwunden werden, welche kleiner ist als die Haftreibung, wodurch die Belastung der Transplantatoberfläche weiter reduziert wird.

Alternativ zu den bereits genannten Fluiden, insbesondere den Flüssigkeiten, kann das Puffermedium als weicher Kunststoff oder als schwammartiger Kunststoff ausgebildet sein, dessen Elastizitätsmodul kleiner ist als der Elastizitätsmodul des weichsten Teils des Transplantats bzw. Implantats, wobei der Kunststoff ggf. mit einer Flüssigkeit gefüllt sein kann. Aufgrund des kleineren Elastizitätsmoduls kann sich das Puffermedium zunächst optimal an die Form der Oberfläche des Transplantats anpassen und überträgt anschliessend (nach Erreichen einer bestimmten Kompression des Puffermediums) die Kräfte optimal auf die Transplantatoberfläche. Als Puffermedium kommt z.B. auch ein mit Fluid, insbesondere Flüssigkeit, gefüllter Ballon in Frage.

Bei einer bevorzugten Weiterbildung des Instrumentensatzes ist auch das Instrument zur Entnahme des Transplantats bzw. Implantats gemäss einem der vorstehend erläuterten Ausführungsbeispiele ausgebildet. Insbesondere kann es so sein, dass das Instrument oder zumindest Teile davon sowohl für die Entnahme (Extraktion) des Transplantats als auch für das Einbringen (Implantation) des Transplantats verwendet werden.

Die Erfindung ist anwendbar in einem Verfahren zur Transplantation eines osteochondralen Transplantats bzw. zur Implantation eines entsprechenden in vitro hergestellten Implantats, bei welchem zunächst an der Implantationsstelle eine Vertiefung zur Aufnahme des Transplantats bzw. Implantats erstellt wird, sodann an einer Erntestelle das Transplantat entnommen wird bzw. das Implantat aus einem entsprechenden Behältnis entnommen wird, und bei schliesslich das Transplantat bzw. Implantat in die an der Implantationsstelle erstellte Vertiefung eingebracht wird. Bei diesem Verfahren wird ein Instrument oder ein Instrumentensatz gemäss einem der vorstehend erläuterten Ausführungsbeispiele verwendet.

Bei einer vorteilhaften Ausgestaltung dieses Verfahrens wird beim Einbringen des im Kanal des Instruments befindlichen Transplantats bzw. Implantats in die Vertiefung hinein von der proximalen Seite her zunächst das Puffermedium und anschliessend der Stempel in den Kanal hinein eingebracht, sodass das eingeschlossene Puffermedium beim Vorschieben des Stempels in distaler Richtung das Transplantat bzw. Implantat aus dem Instrument heraus in die Vertiefung hinein eintreibt. Der operierende Arzt kann also zunächst das Instrument mitsamt dem im Kanal des Instruments befindlichen Transplantat in die gewünschte Position bringen, anschliessend das Puffermedium einbringen (z.B. die erwähnte Ringer-Lösung) und schliesslich den Stempel aufsetzen und vorschieben, sodass mit Hilfe des auf das Puffermedium ausgeübten Drucks dann das Transplantat in die Vertiefung hinein eingetrieben wird.

Weitere vorteilhafte Ausgestaltungen ergeben sich aus der nachfolgenden Beschreibung von Ausführungsbeispielen der Erfindung mit Hilfe der Zeichnung. Es zeigen, teilweise schematisch und/oder im Schnitt:

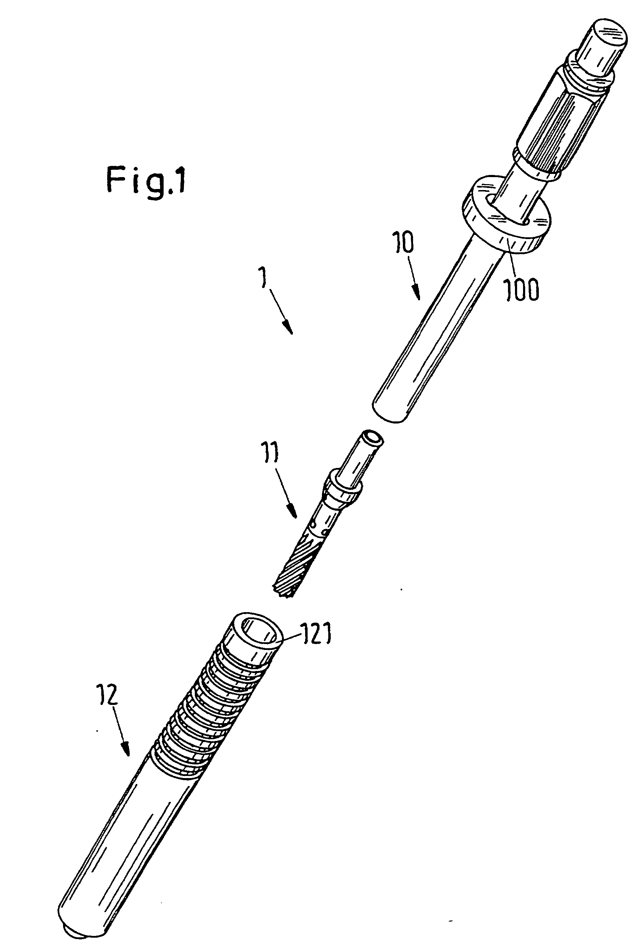

- Fig. 1: ein Ausführungsbeispiel eines Instruments zum Erstellen einer Gewebesäule (Peg) an einer Erntestelle in perspektivischer Explosionsdarstellung,
- Fig. 2: das Instrument aus Fig. 1 in zusammgesetztem Zustand im Schnitt,
- Fig. 3: das Detail III (distales Ende) aus Fig. 2 in vergrösserter Darstellung,
- Fig. 4: ein Ausführungsbeispiel eines Extraktors zur Entnahme der an der Erntestelle erstellten Gewebesäule (Peg) in zusammengesetztem Zustand in perspektivischer Darstellung,
- Fig. 5: das Ausführungsbeispiel des Extraktors gemäss Fig. 4 im Schnitt,
- Fig. 6: das Detail VI (distales Ende) des Extraktors aus Fig. 5 in vergrösserter Darstellung,
- Fig. 7: ein Ausführungsbeispiel eines Instruments zum Einbringen der Gewebesäule (Peg) an der lmplantationssteile in perspektivischer auseinandergezogener Darstellung,
- Fig. 8: einen Ausschnitt des Instruments aus Fig. 7 im Schnitt, welcher die Funktionsweise des Intruments verdeutlicht
und
- Fig. 9: ein Ausführungsbeispiel des distalen Endes einer Hülse, wie sie im Zusammenhang mit einem Instrument gemäss Fig. 8 verwendet werden kann.

In dem Ausführungsbeispiel eines Instruments 1 zur Erstellung einer Gewebesäule an einer Erntestelle erkennt man eine Aufnahmestück 10 zur Aufnahme eines Hohlbohrers oder Hohlfräsers 11 sowie eine Hülse 12, welche an ihrem distalen Ende mit einer Schneide 120 versehen ist. Ist der Hohlfräser 11 in dem Aufnahmestück aufgenommen und fest mit diesem verbunden, so wird er in die Hülse 12 eingeführt. Das Aufnahmestück 10 ist mit einem Anschlagring 100 versehen, welcher an einer beliebigen Stelle aussen auf dem Aufnahmestück arretiert werden kann, z.B. mit Hilfe einer Madenschraube (nicht dargestellt). Beim Einführen des Aufnahmestücks 10 mit dem Hohlfräser 11 in die Hülse 12 hinein wird das Einführen des Aufnahmestücks 10 durch den Anschlagring 100 begrenzt, der gegen die proximale Endfläche 121 der Hülse 12 stösst.

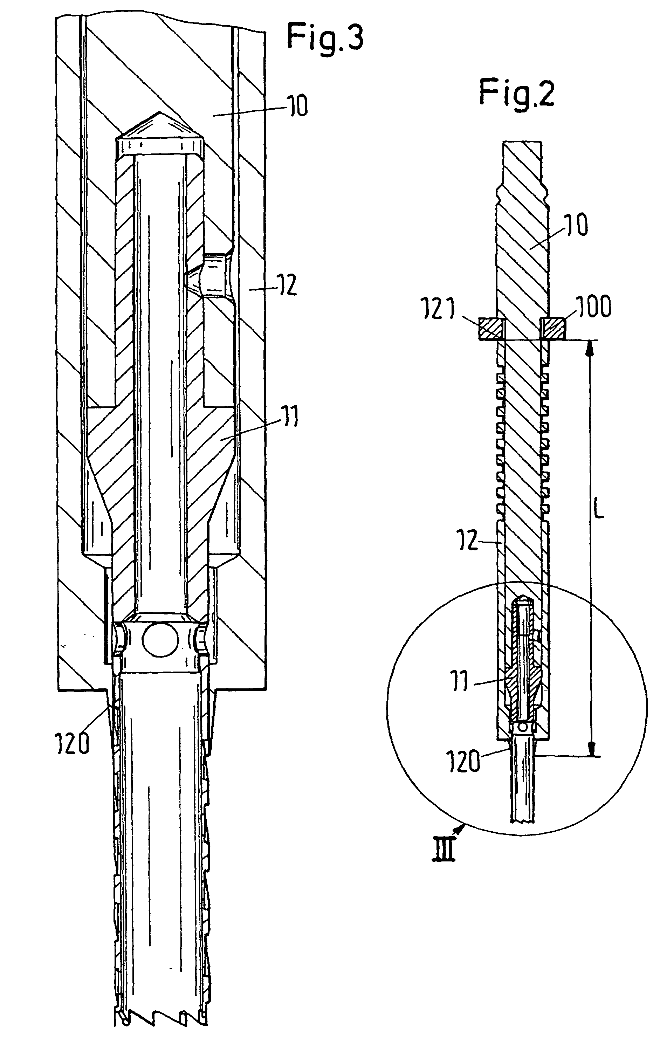

Fig. 2 zeigt das vollständig bis zum Anschlagen des Anschlagrings 100 in die Hülse 12 eingetauchte Aufnahmestück 10 mitsamt dem im Aufnahmestück 10 aufgenommenen und dort fixierten Hohlfräser 11 - der Hohlfräser 11 kann z.B. mit einer Madenschraube (nicht dargestellt) oder einem sonstigen geeigneten Befestigungsmittel in dem Aufnahmestück 10 befestigt sein. Die Distanz L zwischen der proximalen Endfläche 121 der Hülse 12 und dem distalen Ende der Schneide 120 bezeichnet die Länge der Hülse 12 inklusive der Schneide 120. Wenn nun der Hohlfräser 11 stets in derselben Stellung relativ zum Aufnahmestück 10 in diesem befestigt wird (was in der Regel durch die Art der Fixierung zwingend vorgegeben ist), so legt die Position des Anschlagrings 100 auf dem Aufnahmestück fest, wie tief der Hohlfräser 11 in das Gewebe (z.B. den Knochen) eindringen kann, die Position des Anschlagrings 100 bestimmt also die maximale Länge der zu entnehmenden Gewebesäule (Peg). Dabei ist zu beachten, dass auch die Länge der Schneide 120 hierbei eine Rolle spielt. Die Hülse 12 wird nämlich zuerst in den Knorpel eingeschlagen, bis die distale Endfläche 122 der Hülse 12 auf dem Knorpel aufliegt, also bis die Schneide 120 vollständig in den Knorpel eingedrungen ist. Dann kann die Hülse 12 nicht mehr auf dem elastischen Knorpel verrutschen und der Hohlfräser 11 wird optimal geführt.

Auf der Aussenwand des Aufnahmestücks können Markierungen vorgesehen sein (nicht dargestellt), welche dem operierenden Arzt anzeigen, aus welcher Position des Anschlagrings 100 welche Länge des Pegs resultiert, vorausgesetzt der Hohlfräser 11 wird bis zum Anschlagen des Anschlagrings 100 gegen die proximale Endfläche 121 der Hülse 12 vorgetrieben. Diese Stellung, bei welcher der Anschlagring 100 gegen die proximale Endfläche 121 der Hülse 12 anschlägt, ist in Fig. 2 dargestellt. In Fig. 3 ist das Detail III - das distale Ende - noch einmal vergrössert dargestellt.

Nach Beendigung des Fräsens mit Hilfe des Hohlfräsers 11 steht also an der Erntestelle ein Peg, der an seiner Basis (am Boden des Pegs) noch mit dem spongiösen Knochen verbunden ist. Dieser Peg, der mit einer gesunden Knorpelschicht überzogen ist, muss nun entnommen werden.

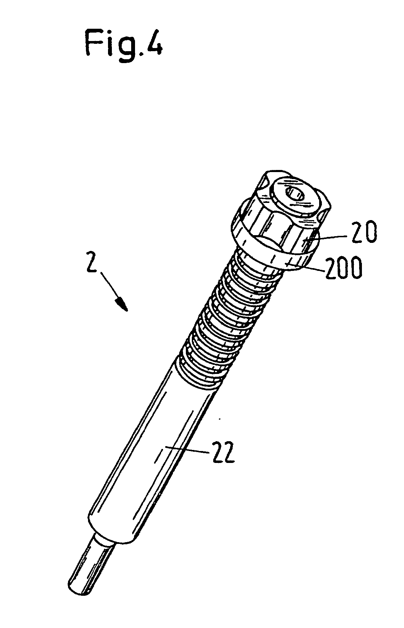

Dies erfolgt mit Hilfe eines Entnahmeinstuments 2 (Fig. 4), das eine Hülse 22 umfasst, welche mit der zuvor beschriebenen Hülse 12 zur Erstellung des Pegs mit Hilfe des Hohlfräsers identisch sein kann und es in der Regel auch ist. Die Hülse 12 bzw. 22 kann also an Ort und Stelle verbleiben, während das Aufnahmestück 10 mitsamt dem Hohlfräser 11 nach der Erstellung der Pegs, der an seiner Basis noch mit dem spongiösen Knochen verbunden ist, aus der Hülse 12 entnommen wird.

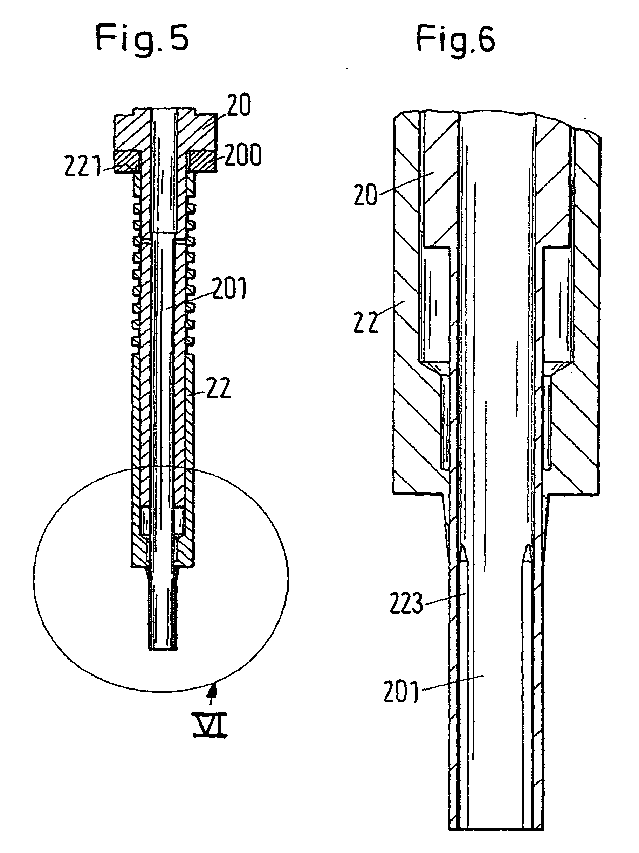

Das Entnahmeinstrument 2 umfasst neben der Hülse 22 noch den eigentlichen Extraktor 20, welcher in Fig. 5 ebenfalls mit einem Anschlagring 200 versehen ist, welcher das Eintreiben des Extraktors 20 in die vorgefräste Vertiefung um den Peg herum begrenzt, sobald nämlich der Anschlagring 200 gegen die proximale Endfläche 221 der Hülse 22 anschlägt. Der Extraktor 20 weist im Bereich seines distalen Endes von seiner Innenwand nach innen abstehende Finnen 223 auf. Diese Finnen 223 graben sich beim Eintreiben des Extraktors 20 in den Peg ein und ermöglichen es, den Peg durch eine Drehbewegung des Extraktors 20 an seiner Basis abzuscheren. Nach dem Abscheren des Pegs befindet sich dieser im Kanal 201 des Extraktors 20.

Auch hier ist es wieder so, dass durch Markierungen auf der Aussenwand des Extraktors dem operierenden Arzt angezeigt werden kann, welche Länge der Peg bei welcher Position des Anschlagrings 200 aufweist. Dabei wird wieder vorausgesetzt, dass die Schneide der Hülse 22 so weit in den Knorpel eingetrieben ist, dass die distale Endfläche 220 der Hülse 22 auf dem Knorpel aufliegt. Dadurch wird - wie bereits erwähnt - auch ein Abrutschen der Hülse 22 auf dem Knorpel verhindert, sodass der Extraktor 20 optimal in der Hülse 22 geführt wird. Fig. 6 zeigt noch einmal in einer vergrösserten Darstellung das Detail VI der Fig. 5, nämlich das distale Ende des Entnahmeinstruments 2.

Der entnommene Peg befindet sich nun im Kanal 201 des Extraktors 20 und muss an die Implantationsstelle (nicht dargestellt) transportiert werden, wo er in eine vorbereitete Sacklochbohrung eingetrieben wird. Dabei ist der Durchmesser der Sacklochbohrung an der Implantationsstelle (Defektstelle) in der Regel geringfügig kleiner als der Durchmesser des an der Erntestelle entnommenen Pegs, sodass ein Pressitz des Pegs in der Sacklochbohrung entsteht.

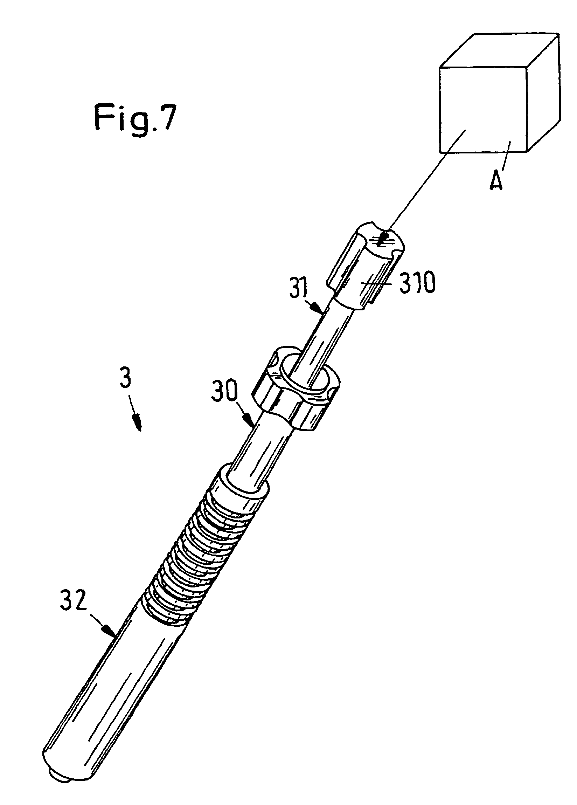

Fig. 7 zeigt ein Ausführungsbeispiel eines Instruments 3 zum Einbringen des Pegs in die an der lmplantationsstelle erstellte Sacklochbohrung. Das Instrument umfasst einen Stempel 31, ein Instrument 30, in welchem der Peg angeordnet ist, welches mit dem zuvor beschriebenen Extraktor 20 identisch sein kann, sowie eine Hülse 32, in welche das Instrument bzw. Extraktor 30 eingeführt werden kann. Der Stempel 31 weist an seinem proximalen Ende ein Anschlussstück 310 auf, welches von einem Antrieb A aufgenommen werden und von diesem angetrieben bzw. vorgetrieben werden kann. Auf die Art und Weise, wie dies erfolgt, wird weiter unten noch genauer eingegangen.

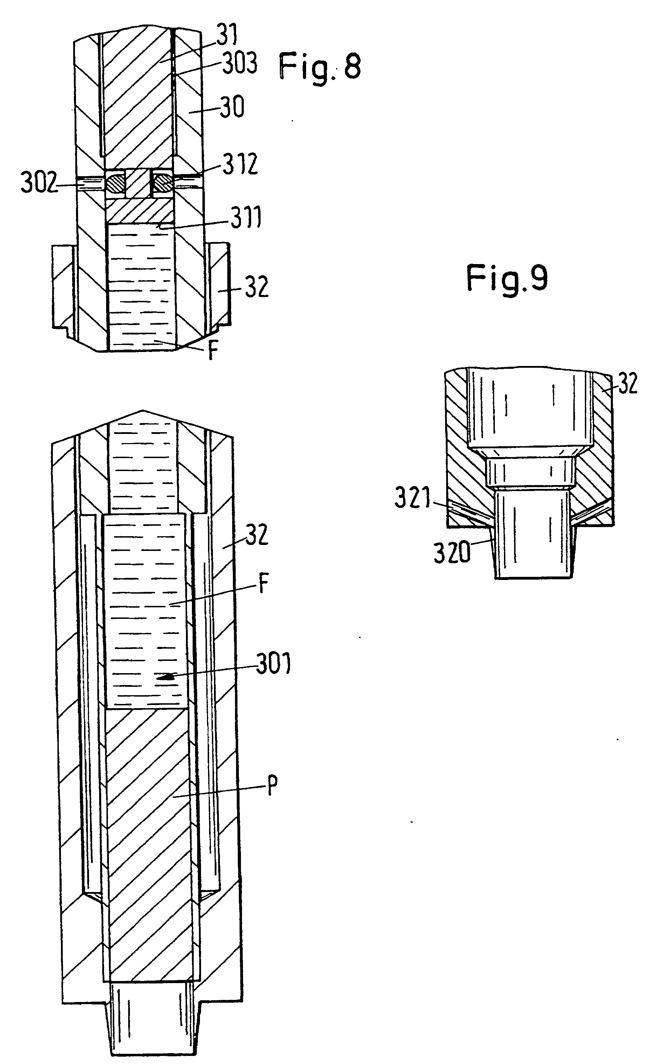

Fig. 8 zeigt in einem Ausschnitt aus Fig. 7 zwei wesentliche Details, welche die Funktionsweise der Vorrichtung verdeutlichen. Man erkennt die Hülse 32, in welcher der Extraktor 30 vollständig vorgeschoben ist. Im Kanal 301 des Extraktors 30 ist der Peg P am distalen Ende des Kanals 301 angeordnet. Proximal des Pegs P ist im Kanal ein Puffermedium angeordnet, hier in Form einer Ringer-Lösung F, wie sie typischerweise im Operationssaal als Spüllosung zum Einsatz kommt. Alternativ kann auch eine Natriumchloridlösung (Kochsalzlösung) zum Einsatz kommen oder ein weicher oder schwammartiger Kunststoff, dessen Elastizitätsmodul kleiner ist als der Elastizitätsmodul des Knorpels (weichster Teil des Transplantats). Gegebenenfalls kann der Kunststoff mit einer Flüssigkeit gefüllt sein. Für das Ausführungsbeispiel gemäss Fig. 8 wird aber im folgenden von einer Flüssigkeit, nämlich der bereits erwähnten Ringer-Lösung F, als Puffermedium ausgegangen.

Der Stempel 31 ist in seinem distalen Endbereich, also nahe der Stirnfläche 311, die dem Peg P zugewandt ist, mit einem O-Ring 312 versehen. Ferner erkennt man, dass im Kanal 301 in dem Raum zwischen dem proximalen Ende des Pegs P und den Öffnungen 302 in der Wand des Extraktors 30 die Flüssigkeit F eingefüllt ist. Wenn nun der O-Ring 311 in distaler Richtung an den Öffnungen 302 vorbeigleitet, dichtet er den Kanal 301 ab und verhindert, dass beim Vorschieben des Stempels 31 in distaler Richtung die Ringer-Lösung F in proximaler Richtung zurückströmen kann. Auf diese Weise wird also beim Vorschieben oder Vortreiben des Stempels 31 der Peg P aus dem distalen Ende der Hülse 32 heraus in die Sacklochbohrung an der Implantationsstelle (nicht dargestellt) hinein gedrückt, ohne dass ein körperlicher Kontakt zwischen dem Stempel und dem Peg besteht.

Das Anschlusstück 310 kann dabei einen Sicherheitsanschlag für das Vorschieben bzw. Vortreiben des Stempels 31 bilden. Hierfür kann auch ein Anschlagring vorgesehen sein, wie er weiter oben schon beschrieben ist. Diese Anschläge sind aber im Prinzip "Sicherheitsanschläge", eine bevorzugte Art eines nicht-mechanischen "Anschlags" wird weiter unten anhand von Fig. 9 noch erläutert.

Was das Vorschieben bzw. Vortreiben des Stempels 31 in distaler Richtung betrifft, so ist bereits weiter oben festgestellt worden, dass ein kontinuierliches Vorschieben des Pegs P vorteilhaft ist (nur einmaliges Überwinden der Haftreibung und dann nur noch der Gleitreibung). Zu diesem Zweck kann der Antrieb A das Ansschlussstück 310 und damit den Stempel 31 kontinuierlich drehen. Der Vorschub des Stempels 31 kann derart bewirkt werden, dass distal zu den Öffnungen 302 der Extraktor 30 auf seiner Innenwand mit einem Gewinde 303 versehen ist und der Stempel 31 in einem entsprechenden Bereich ebenfalls ein entsprechendes Gewinde auf seiner Aussenwand aufweist (nicht dargestellt). Bei einem kontinuierlichen Drehen des Stempels 31 wird der Stempel dann entsprechend der Steigung des Gewindes in distaler Richtung vorgeschoben. Wenn das distale Ende des Gewindes auf der Aussenwand des Stempels 31 dann das distale Ende des Gewindes 303 auf der Innenwand des Extraktors 30 erreicht, kann das weitere Vortreiben des Stempels auch auf diese Weise verhindert werden. Auch dies ist eine Art und Weise der Realisierung eines mechanischen "Sicherheistanschlags", eine bevorzugte Art eines nicht-mechanischen "Anschlags" wird im folgenden erläutert.

Dazu muss der operierende Arzt eine Möglichkeit haben, zu erkennen, dass der Peg P bis zu einem gewünschten Mass aus der Hülse 32 ausgetrieben worden ist. Wie dies beispielsweise möglich ist, geht aus Fig. 9 hervor, in welcher eine Art und Weise dargestellt ist, wie das distale Ende der Hülse 32 ausgebildet sein kann. In der Wand der Hülse 32 sind unmittelbar anschliessend an das proximale Ende der Schneide 320 Durchtrittsöffnungen 321 vorgesehen. Diese Durchtrittsöffnungen 321 ermöglichen ein Austreten der Ringer-Lösung F, sobald das proximale Ende des Pegs P die Durchtrittsöffnungen 321 freigibt. Damit wird aber bei einem weiteren Vorschieben des Stempels 31 praktisch keine Kraft mehr auf den Peg P ausgeübt, weil die Ringer-Lösung F bei einem weiteren Vorschieben des Stempels die Flüssigkeit durch die Durchtrittsöffnungen 321 hindurch herausgedrückt wird (nicht-mechanischer "Anschlag"). Gegebenenfalls könnte man die Öffnungen 321 derart ausbilden, dass noch eine geringe Kraftübertragung auf den Peg P möglich ist, um ihn noch um eine geringe Strecke vortreiben zu können.

Das Verfahren zur Transplantation bzw. Implantation kann also folgendermassen ablaufen: Zunächst erstellt der Arzt an der Implantationsstelle (Defektstelle) eine Vertiefung (z.B. Sacklochbohrung) zur Aufnahme des Transplantats oder Implantats. Dies kann mit Hilfe eines konventionellen chirurgischen Knochenbohrers erfolgen, der ggf. auch in einer Hülse geführt sein kann. Dabei ist klar, dass der Durchmesser dieser Sacklochbohrung geringfügig kleiner ist als der Durchmesser des Pegs P, damit es bei der Implantation des Pegs zu einem Pressitz des Pegs P in der Sacklochbohrung kommt.

Nach der Erstellung der Sacklochbohrung an der Implantationsstelle wird an der Erntestelle z.B. mit Hilfe des Instruments 1, insbesondere mit Hilfe des Hohlfräsers 11, ein Peg P erstellt, der an seiner Basis noch mit dem Knochen verbunden ist. Anschliessend wird dieser Peg mit Hilfe des Instruments 2, insbesondere mit Hilfe des Extraktors 20, von seiner Basis getrennt und entnommen. Im Falle eines in vitro hergestellten Implantats wird der Peg aus einem entsprechenden Behältnis entnommen. Der entnommene Peg P wird dann zur Implantationsstelle transportiert. Dort kann mit Hilfe des Instruments 3 der Peg P implantiert werden. Dies kann insbesondere so erfolgen, dass vor dem Einbringen des Pegs P zunächst ein Puffermedium - wie die erwähnte Ringer-Lösung F - von der proximalen Seite her in den Kanal 301 des Extraktors 30 eingebracht werden kann. Anschliessend kann der Stempel 31 von der proximalen Seite her in den Kanal 301 eingebracht werden. Beim Vorschieben bzw. Vortreiben des Stempels 31 (z.B. mittels des Antriebs 31 oder aber auch von Hand) treibt dann die eingeschlossene Ringer-Lösung F den Peg P aus dem Instrument 3 heraus in die Sacklochbohrung an der Implantationsstelle ein. Es ist klar, dass sich die oben beschriebenen Instrumente, insbesondere ein ganzer Satz von solchen Instrumenten, die für eine derartige Operation benötigt werden, besonders eignen.

## Patentansprüche

1. Instrument (2,3) zum Einbringen eines an einer Erntestelle entnommenen osteochondralen Transplantats (P) oder eines entsprechenden in vitro hergestellten Implantats an einer Implantationsstelle, welches Instrument einen Kanal (201,301) aufweist, in welchem das Transplantat bzw. Implantat aufgenommen wird, sowie einen Stempel (31) zum Herausdrücken des Transplantats (P) bzw. Implantats aus dem distalen Ende des Kanals heraus in eine an der Implantationsstelle vorbereitete Vertiefung hinein, dadurch gekenhzeichnet, dass mechanische oder nicht-mechanische Anschlagmittel vorgesehen sind, die derart ausgebildet sind, dass zwischen der dem Transplantat (P) bzw. Implantat zugewandten Stirnfläche (311) des Stempels (31) und einer Ebene, die von einer als Auflagefläche dienenden distalen Endfläche einer den Kanal umgebenden Hülse (32) definiert ist, in jeder Arbeitsstellung des Stempels (31) ein Raum zur Aufnahme eines Puffermediums (F) mit einer hohen Formanpassungsfähigkeit und einer Elastizität vorhanden ist, die so bemessen ist, dass beim Herausdrücken des Transplantats (P) bzw. Implantats kein körperlicher Kontakt zwischen dem Stempel (31) und dem Transplantat (P) bzw. Implantat besteht.

2. Instrument nach Anspruch 1, **dadurch gekennzeichnet, dass** der Stempel (31) mit mechanischen Anschlagmitteln (310,303) versehen ist, welche eine Endstellung des Stempels (31) definieren und ein weiteres Vorschieben des Stempels (31) über die Endstellung hinaus verhindern.

3. Instrument nach Anspruch 1 oder 2, **dadurch gekennzeichnet, dass** das Puffermedium ein Fluid ist, insbesondere eine Flüssigkeit (F), und dass der Stempel (31) mit einem Dichtmittel (312) versehen ist, welches den Kanal (301) abdichtet und beim Vorschieben des Stempels (31) in distaler Richtung ein Zurückströmen von Fluid in proximaler Richtung verhindert.

4. Instrument nach Anspruch 3, **dadurch gekennzeichnet, dass** das Dichtmittel als O-Ring (312) ausgebildet ist, welcher im distalen Endbereich des Stempels (31) angeordnet ist, und zwar proximal zu der dem Transplantat (P) zugewandten Stirnfläche (311) des Stempels (31).

5. Instrument nach Anspruch 4, **dadurch gekennzeichnet, dass** die Hülse (32) an ihrem distalen Ende mit einer Schneide (320) versehen ist, und dass in der Wand der Hülse (320) unmittelbar anschliessend an das proximale Ende der Schneide (320) als nicht-mechanische Anschlagmittel dienende Durchtrittsöffnungen (321) vorgesehen sind, welche ein Austreten des Fluids (F) ermöglichen, sobald beim Herausdrücken des Transplantats (P) bzw. Implantas das proximale Ende des Transplantats (P) bzw. Implantats die Durchtrittsöffnungen (321) freigibt.

6. Instrument nach einem der vorangehenden Ansprüche, **dadurch gekennzeichnet, dass** der Stempel (31) an seinem proximalen Ende mit einem Antrieb (A) koppelbar ist, welcher einen kontinuierlichen Vorschub des Stempels (31) bewirkt.

7. Instrument nach einem der vorangehenden Ansprüche, **dadurch gekennzeichnet, dass** das Puffermedium als weicher Kunststoff oder als schwammartiger Kunststoff ausgebildet ist, dessen Elastizitätsmodul kleiner ist als der Elastizitätsmodul des weichsten Teils des Transplantats bzw. Implantats, wobei der Kunststoff ggf. mit einer Flüssigkeit gefüllt sein kann.

8. Instrumentensatz für die Transplantation eines osteochondralen Transplantats oder für die Implantation eines entsprechenden in vitro hergestellten Implantats, welcher ein Instrument (1,2) zur Entnahme des Transplantats an einer Erntestelle umfasst bzw. zur Entnahme des in vitro hergestellten Implantats aus einem entsprechenden Behältnis, welcher ferner ein Instrument zur Erstellung einer Vertiefung an der Implantationsstelle umfasst, und welcher ein Instrument (2,3) zum Einbringen des entnommenen Transplantats an der Implantationsstelle umfasst, **dadurch gekennzeichnet, dass** das Instrument (2,3) zum Einbringen des Transplantats bzw. Implantats gemäss einem der vorangehenden Ansprüche ausgebildet ist.

9. Instrumentensatz nach Anspruch 8, **dadurch gekennzeichnet, dass** das Instrument (1,2) zur Entnahme des Transplantats bzw. Implantats gemäss einem der Ansprüche 1 bis 7 ausgebildet ist.

## Claims

1. Instrument (2, 3) for the introduction of an osteochondral transplant (P) which is removed from a harvest location, or of an implant which is correspondingly prepared in vitro, at an implantation location, said instrument having a passage (201, 301) in which the respective transplant or implant is received and a piston (31) for the pressing of the transplant (P) or implant from the distal end of the passage into a recess which is prepared at the implant location, **characterised in that** mechanican abutments or non-mechanical abutments are provided and designed such that a space for the reception of a buffer medium (F) with a high conformability and an elasticity which is dimensioned in such a manner that no bodily contact between the piston (31) and the transplant (P) or the implant exists during the pressing out of the transplant (P) or the implant is present in every operating position of the piston (31) between the end surface (311) of the piston (31) facing the transplant (P) or the implant and a plane which is defined by a distal end face of a sleeve (32) surrounding the passage serving as a contact surface.

2. Instrument in accordance with claim 1, **characterised in that** the piston (31) is provided with mechanical abutment means (310, 303) which define an end position of the piston (31) and prevent a further pushing forward of the piston (31) beyond the end position.

3. Instrument in accordance with claim 1 or claim 2, **characterised in that** the buffer medium is a fluid, in particular a liquid (F); and **in that** the piston (31) is provided with a sealing means (312) which seals off the passage (301) and prevents a backward flow of fluid in the proximal direction during the pushing forward of the piston (31) in the distal direction.

4. Instrument in accordance with claim 3, **characterised in that** the sealing means is made as an O ring (312) which is arranged in the distal end region of the piston (31), and indeed proximal to the end surface (311) of the piston (31) facing the transplant (P).

5. Instrument in accordance with claim 4, **characterised in that** a sleeve (32) is provided with a cutting edge (320) at its distal end; and **in that** passage openings (321) which serve as non-mechanical abutment means are provided directly following the proximal end of the cutting edge (320) in the wall of the sleeve (320), which enable an emergence of the fluid (F) as soon as the proximal end of the transplant (P) or of the implant respectively releases the passage openings (321) during the pressing out of the transplant (P) or of the implant respectively.

6. Instrument in accordance with any one of the preceding claims, **characterised in that** the piston (31) can be coupled at its proximal end to a drive (A) which effects a continuous pushing forward of the piston (31).

7. Instrument in accordance with any one of the preceding claims, **characterised in that** the buffer medium is made as soft plastic or as sponge-like plastic, the elastic module of which is less than the elastic module of the softest part of the transplant or of the implant respectively, with it being possible for the plastic optionally to be filled with a liquid.

8. Instrument set for the transplant of an osteochondral transplant or for the implant of an implant which is correspondingly prepared in vitro, comprising an instrument (1, 2) for the removal of the transplant at a harvest location or, respectively, for the removal of the implant which is produced in vitro from a corresponding container, further comprising an instrument for the production of a recess at the implant location, and comprising an instrument (2, 3) for the introduction of the removed transplant at the implant location, **characterised in that** the instrument (2, 3) is designed for the introduction of the transplant or the implant in accordance with any one of the preceding claims.

9. Instrument set in accordance with claim 8, **characterised in that** the instrument (1, 2) for the removal of the transplant or the implant is designed in accordance with any one of the claims 1 to 7.

## Revendications

1. Instrument (2, 3) pour l'introduction, dans une zone d'implantation, d'un greffon ostéochondral (P) prélevé dans une zone donneuse, ou d'un implant correspondant généré *in vitro,* lequel instrument comprend un canal (201, 301) dans lequel le greffon ou l'implant est respectivement reçu, ainsi qu'un bloc presseur (31) pour expulser respectivement ledit greffon (P) ou ledit implant, depuis l'extrémité distale du canal jusque dans un renfoncement apprêté dans la zone d'implantation, **caractérisé en ce que** des moyens de butée mécaniques ou non mécaniques sont prévus et réalisés de manière à réserver dans chaque position de travail du bloc presseur (31), entre la face extrême (311) dudit bloc presseur (31) respectivement tournée vers le greffon (P) ou l'implant, et un plan défini par une surface extrême distale d'une douille (32) ceinturant le canal, remplissant la fonction d'une surface d'appui, un espace destiné à recevoir un agent tampon (F) pourvu d'une haute faculté d'adaptation de forme et d'une élasticité calculée de façon telle qu'il n'existe, au stade de l'expulsion respective du greffon (P) ou de l'implant, aucun contact corporel entre le bloc presseur (31) et ledit greffon (P) ou ledit implant.

2. Instrument selon la revendication 1, **caractérisé par le fait que** le bloc presseur (31) est doté de moyens de butée mécaniques (310, 303) qui définissent une position extrême dudit bloc presseur (31) et interdisent une poursuite de l'avance dudit bloc presseur (31) au-delà de ladite position extrême.

3. Instrument selon la revendication 1 ou 2, **caractérisé par le fait que** l'agent tampon est un fluide, en particulier un liquide (F), et **par le fait que** le bloc presseur (31) est équipé d'un moyen d'étanchement (312) qui assure l'étanchéité du canal (301) et empêche, lors de l'avance dudit bloc presseur (31) dans la direction distale, un reflux de fluide dans la direction proximale.

4. Instrument selon la revendication 3, **caractérisé par le fait que** le moyen d'étanchement est réalisé sous la forme d'un joint torique (312) disposée dans la région extrême distale du bloc presseur (31) et occupant, plus précisément, une position proximale vis-à-vis de la face extrême (311) dudit bloc presseur (31) qui est tournée vers le greffon (P).

5. Instrument selon la revendication 4, **caractérisé par le fait que** la douille (32) est dotée d'un tranchant (320) à son extrémité distale, et **par le fait que** des orifices de passage (321) remplissant la fonction de moyens de butée non mécaniques prévus dans la paroi de ladite douille (32), dans la continuité directe de l'extrémité proximale dudit tranchant (320), autorisent une sortie du fluide (F) aussitôt que l'extrémité proximale respective du greffon (P) ou de l'implant dégage lesdits orifices de passage (321) lors de l'expulsion respective dudit greffon (P) ou dudit implant.

6. Instrument selon une des revendications précédentes, **caractérisé par le fait que** le bloc presseur (31) peut être accouplé, par son extrémité proximale, à un entraînement (A) provoquant une avance continue dudit bloc presseur (31).

7. Instrument selon une des revendications précédentes, **caractérisé par le fait que** l'agent tampon est conçu comme une matière plastique tendre ou une matière plastique du type éponge dont le module d'élasticité est plus petit que le module d'élasticité de la partie la plus molle du greffon ou de l'implant, ladite matière plastique pouvant être éventuellement emplie d'un liquide.

8. Jeu d'instruments pour la transplantation d'un greffon ostéochondral ou pour l'implantation d'un implant correspondant généré *in vitro*, comprenant un instrument (1, 2) pour prélever le greffon dans une zone donneuse ou pour prélever respectivement, d'un réceptacle correspondant, l'implant généré *in vitro* ; comprenant, par ailleurs, un instrument pour façonner un renfoncement dans la zone d'implantation, et comprenant un instrument (2, 3) pour introduire dans la zone d'implantation le greffon prélevé, **caractérisé par le fait que** l'instrument (2, 3) d'introduction respective du greffon ou de l'implant est réalisé conformément à l'une des revendications précédentes.

9. Jeu d'instruments selon la revendication 8, **caractérisé par le fait que** l'instrument (1, 2) de prélèvement respectif du greffon ou de l'implant est réalisé conformément à l'une des revendications 1 à 7.
